# EUROPÄISCHE PATENTSCHRIFT

(11) **EP 0 570 544 B1**
(45) Veröffentlichungstag und Bekanntmachung des Hinweises auf die Patenterteilung: **19.08.1998**
(21) Anmeldenummer: 92921387.4
(22) Anmeldetag: 22.10.1992
(51) Int. Cl.: A61N 1/40

(54) **VORRICHTUNG ZUM BEWIRKEN DES TRANSPORTS VON PROTONEN AUS INTRAKORPORALEN ELEKTROLYT-FLÜSSIGKEITEN**
DEVICE FOR CAUSING PROTONS TO MIGRATE OUT OF ELECTROLYTIC INTERNAL BODY FLUIDS
DISPOSITIF DESTINE A FAIRE SORTIR DES PROTONS DE LIQUIDES ELECTROLYTIQUES INTRACORPORELS

(30) Priorität: 24.10.1991 CH 3110/91
(43) Veröffentlichungstag der Anmeldung: 24.11.1993
(73) Patentinhaber: LARSEN, Eric, 8201 Schaffhausen (CH)
(72) Erfinder: LARSEN, Eric, 8201 Schaffhausen (CH)
(74) Vertreter: Patentanwälte Möll und Bitterich
(86) Internationale Anmeldenummer: CH9200215
(87) Internationale Veröffentlichungsnummer: WO9307932

(56) Entgegenhaltungen:
- EP-A- 0 132 051
- EP-A- 0 291 569
- EP-A- 0 330 797
- EP-A- 0 407 057
- WO-A-93/00960
- US-A- 4 667 677

## Beschreibung

Die Erfindung betrifft eine Vorrichtung zum Bewirken des Transports von Protonen aus intrakorporalen Elektrolyt-Flüssigkeiten in die sie umgebenden Gefäßwände.

Die bekanntesten Vertreter der intrakorporalen Elektrolyt-Flüssigkeiten bei Menschen und Tieren sind das Blut und die Lymphe.

Das Blut wird vom Herzen unter Druck, dem sogenannten Blutdruck, in geschlossenen Gefäßen gepumpt, und zwar zum Herzen hin in Venen und vom Herzen weg in Arterien bis hin zu den Kapillaren, die in das Gewebe münden. Das Blut ist im Körper das universelle Transportmittel für Sauerstoff, Kohlendioxid, Wasser, Salze und andere Elektrolyte, Nährstoffe, Stoffwechselabbauprodukte, Wärme, katalytisch wirkende Stoffe wie Hormone und Enzyme, Antikörper, Wundverschlußstoffe usw. Blut läßt sich auftrennen in feste Bestandteile und in flüssiges Blutplasma. Das Blutplasma enthält Blutzucker, die Blutfette, gelöste Nähr- und Abfallstoffe, Fibrinogen, Globuline und andere Eiweißstoffe, die Blutgruppensubstanzen und Rhesusfaktoren sowie gegebenenfalls den Blutalkohol. Die festen, geformten Bestandteile des Blutes sind die Blutkörperchen. Bei diesen unterscheidet man Leukocyten, Thrombocyten und Erythrocyten. Die letzteren bestehen zu 79 % aus Hämoglobin und sind für den Sauerstoff-Kohlendioxid-Transport verantwortlich.

Seinem Fließverhalten nach ist das Blut eine nicht-Newtonsche Flüssigkeit, eher vergleichbar einer Emulsion als einer Suspension. Der pH-Wert beträgt ca. 7,38. Die relative Dielektrizitätskonstante liegt entsprechend dem hohen Wassergehalt bei 80.

Die Lymphe ist eine farblose bis gelbliche Körperflüssigkeit, die aus dem Blutplasma entsteht und durch die Kapillaren in das Gewebe abgegeben wird. Sie umgibt alle Zellen. Sie sammelt sich in Gewebespalten und Hohlräumen. Die Ableitung erfolgt zunächst in kleinen Lymphkapillaren, die sich zu größeren Lymphgefäßen zusammenschließen. Bevor diese wieder in den Blutkreislauf einmünden, passieren sie die Lymphknoten. Die Lymphe gibt Nährstoffe an das Gewebe und transportiert Stoffwechselprodukte aus dem Gewebe ab. Die Lymphe enthält ca. 95 % Wasser.

Von besonderer Bedeutung für die einwandfreie Funktion des menschlichen und tierischen Organismus ist das sogenannte Säure-Basen-Gleichgewicht, das innerhalb enger Grenzen konstant gehalten werden muß, da sonst erhebliche Funktionsstörungen, z. B. des Ionen-Antagonismus, der Sauerstoff-Transportfunktion des Blutes, der Zellmembran-Durchlässigkeit im Gewebe, der Eigenschaften der Enzyme usw., eintreten können. Das Säure-Basen-Gleichgewicht steht in engem Zusammenhang mit dem allgemeinen Elektrolyt-Haushalt des Körpers.

Es ist bekannt, daß an den Blutgefäßen im Koronar- und Halsbereich sogenannte Baro-Rezeptoren sitzen, mit deren Hilfe der Körper den Blutdruck steuert. Es ist bereits bekannt, daß es möglich ist, diese Baro-Rezeptoren mit Hilfe von elektromagnetischen Feldern derart zu beeinflussen, daß der Blutkreislauf aktiviert wird. Es konnte auf diese Weise auch eine Erweiterung der Kapillaren erreicht werden, was zu einer verbesserten Durchblutung der entsprechenden Körperbereiche führte.

Da die Baro-Rezeptoren nur an bestimmten Stellen der Blutgefäße sitzen, sind deren Einflußmöglichkeiten recht begrenzt. Eine verbesserte Ver- und Entsorgung bestimmter Gefäße und Körperbereiche kommt nur indirekt über eine allgemein verbesserte Durchblutung zustande. Eine Beeinflussung des Lymph-Gefäßsystems ist praktisch nicht möglich, da dieses nicht über Baro-Rezeptoren verfügt.

In der älteren, nicht vorveröffentlichten PCT/DE 92/00564 (WO 93/00960) ist ein Gerät beschrieben zum Transport von Ionen, insbesondere Protonen, aus intrakorporalen Flüssigkeiten in und durch die sie umgebenden Gefäßwände und Membranen. Ein Generator speist eine Sendespule mit einer Pulsfolgeserie von 0,5 bis 35 Hz, die mit Grundstrompulsen und Grundpulspausen einer Grundfrequenz von 100 bis 1000 Hz amplitudenmoduliert ist, wobei jedem Grundstrompuls Hochfrequenzpulse mit 10 bis 100 kHz überlagert sein können. Alle Frequenzen und Amplituden sind speziell so gewählt, daß die Grundpulsfolge ohne Polaritätswechsel moduliert ist.

Der vorliegenden Erfindung liegt die Aufgabe zugrunde, eine Vorrichtung der eingangs genannten Art anzugeben, die eine gezielte Verbesserung der Ver- und Entsorgung beliebiger Körperbereiche bei Menschen und Tieren ermöglicht.

Diese Aufgabe wird gelöst durch die Vorrichtung mit den Merkmalen des Anspruchs 1.

Dank der kombinierten Wirkung von Impulsfrequenz, Impulsform mit Polaritätswechsel, Impulsenergie und Sendespulenform ist es möglich, Protonen aus der intrakorporalen Elektrolyt-Flüssigkeit, beispielsweise dem Blut oder der Lymphe, gezielt und unmittelbar in die sie umgebenden Gefäßwände und Membranen einzuschleusen. Dies ist normalerweise nicht möglich, da die Lipide in den Membranen der Blutgefäßwände, die in Kontakt mit dem Blut stehen, eine negative Ladung tragen. Das dadurch ausgelöste Feld übersteigt die thermische Energie und reicht etwa 1000 Å in das Plasma. Die Konzentration von Kationen nahe an der Gefäßoberfläche ist etwa eine Zehnerpotenz höher als in der Plasmaphase. Dadurch ist der lokale pH-Wert um eine Zehnerpotenz niedriger als in der Elektrolyt-Flüssigkeit.

Normalerweise stellt das Oberflächenpotential eine elektrische Barriere dar, die Protonen und Ionen daran hindert, in die Gefäßwand einzudringen. Die Energie, die ein Ion mit einem Radius von 10⁻¹⁰m benötigt, um aus Wasser in ein Lipidmedium überzutreten, beträgt ca. 22,6 eV.

Unter dem Einfluß der erfindungsgemäßen, pulsierenden elektromagnetischen Felder wird in der Elektrolyt-Flüssigkeit eine elektrische Spannung bestimmter Stärke und Richtung induziert. Unter deren Einfluß bewegen sich die Ionen und wegen ihrer höheren Beweglichkeit bevorzugt die Protonen in Richtung auf die Gefäßwände. Aufgrund der dadurch entstehenden Wechselwirkung wird das induzierte Feld auf eine kleine Fläche konzentriert. Dadurch entsteht eine sogenannte Konzentrationspolarisation. Die in der Gefäßwand induzierte Spannung wird verstärkt. Aufgrund der Tatsache, daß die Gefäßwände und insbesondere deren Membranen sehr dünn sind, entstehen sehr hohe Feldstärken, auch wenn die induzierten Spannungen absolut klein bleiben. So führt beispielsweise eine induzierte Spannung von nur 30 mV in einer Membran von 200 nm Dicke zu einer Feldstärke von 150 kV/m.

Es sei hier darauf hingewiesen, daß Feldstärken dieser Größenordnung nur auf induktivem Wege mit elektromagnetischen Feldern, auf keinen Fall auf kapazitivem oder galvanischem Wege mit Elektroden erzielt werden können. Aufgrund der hohen Leitfähigkeit der intrakorporalen Elektrolyt-Flüssigkeiten wie Blut und Lymphe bilden die Gefäße gleichsam einen Faradayschen Käfig, dessen Inneres feldfrei bleibt. Die induktive Erregung dagegen bedient sich gerade der elektrischen Leitfähigkeit der intrakorporalen Elektrolyt-Flüssigkeiten zur Erzeugung der Spannungen und Felder.

Die schon erwähnte negative Ladung der Gefäßwände gegenüber den Elektrolyt-Flüssigkeiten ist auch bedingt durch die Unterschiede der relativen Dielektrizitätskonstanten der Gefäßwände einerseits und der Flüssigkeiten andererseits. Blut und Lymphe haben aufgrund ihres hohen Wassergehaltes relative Dielektrizitätskonstanten in der Größenordnung von 80. Die Gefäßwände besitzen relative Dielektrizitätskonstanten in der Größenordnung von 3 bis 5. Die erfindungsgemäß in den Elektrolyt-Flüssigkeiten induzierten Spannungen und elektrischen Felder sind in der Lage, diese Zeta-Potential genannte Potentialschwelle zu neutralisieren. Dadurch können nun Ionen und insbesondere die beweglichen Protonen verstärkt in die Zell- und Gefäßwände übertreten. Durch die Anreicherung der Protonen in den Zell- und Gefäßwänden kommt es dort zur Ausbildung einer umgekehrt polarisierten Potentialschwelle, die die Protonen und Ionen daran hindert, die Zell- und Gefäßwände wieder zu verlassen.

Durch die Neutralisation des Zeta-Potentials kommt es außerdem zu einer günstigen Veränderung des pH-Wertes, speziell im Bereich der Gefäßwände.

Alle diese Effekte sind besonders wirksam, je kleiner die Durchmesser der Gefäße sind. Sie sind also besonders stark im Bereich der Kapillaren, wo bekanntlich der Austausch des vom Blut mitgeführten Sauerstoffs gegen das von den Zellen abgegebene Kohlendioxid stattfindet.

Neben den bisher beschriebenen Effekten mit Langzeitwirkung haben die elektromagnetischen Felder weitere Wirkungen. Es seien hier nur genannt die Elektrostriktion der Membranen und Gefäßwände, das Ausrichten von polyvalenten Ionenketten, die tangentiale Verschiebung von adsorbierten Gegen-Ionen, die Kraftwirkung auf dielektrische Körper in homogenen und inhomogenen Feldern sowie die Elektroosmose.

Die Grundfrequenz der Grundpulse ist vorzugsweise abgestimmt auf die mechanische Resonanz der Blut-und Lymphgefäße.

Vorzugsweise wechseln die Grundpulse nach jeder halben Pulszeit die Amplitude.

Als optimale Amplitudenform der Grundpulse haben sich symmetrische Dreiecke bewährt.

Vorteilhafterweise werden den Grundpulsen weitere Pulspakete einer erhöhten Frequenz von ca. 10 kHz überlagert. Diese Frequenz ist abgestimmt auf die kapazitive Überleitung durch die Membranen hindurch.

Um die durch die induzierten Spannungen und Felder ausgelösten Effekte optimal wirken zu lassen, benötigt der Organismus bestimmte Pausen. Vorteilhafterweise werden deshalb die Grundpulse in regelmäßigen Folgen ein- und ausgeschaltet, wobei die Ein/Aus-Zeiten bei ca. 0,3 sec. : 0,7 sec. liegen.

Da sich unter der Wirkung der gepulsten elektromagnetischen Felder der Zustand des Organismus verändert, kann durch eine Anpassung der Ein/Aus-Zeiten die Wirkung weiter optimiert werden. Vorteilhafterweise werden die Ein-Aus-Zeiten mittels Biofeedback variiert.

Zu diesem Zweck kann gemäß einer ersten Variante ein Blutdruckmeßgerät an die erfindungsgemäße Vorrichtung anschließbar sein. In diesem Fall erfolgt die Regelung auf einen optimalen Wert des Blutdrucks.

Gemäß einer zweiten Variante kann ein Thermograph anschließbar sein. In diesem Fall erfolgt die Regelung auf eine optimale Erwärmung des gewünschten Körperbereiches durch verbesserte Durchblutung.

Gemäß einer dritten Variante kann ein Pulsmeßgerät anschließbar sein. Damit wird die Erkenntnis ausgewertet, daß bei optimaler Wirkung der gepulsten elektromagnetischen Felder sich der Herzschlag verlangsamt.

Schließlich kann auch eine induktive Fühlerwicklung anschließbar sein, wobei diese vorzugsweise mit der Sendespule auf derselben Trägerplatte angebracht ist. Diese Fühlerwicklung nimmt das von der Sendespule abgestrahlte und vom bestrahlten Organismus phasenverschoben reflektierte Magnetfeld auf. Das aufgenommene Signal wird mit einer geeigneten elektronischen Auswerteschaltung im Gerät selbst im Sinne einer Optimierung ausgewertet.

Form und Konstruktion der Sendespule sind von wesentlicher Bedeutung für eine optimale Wirkung auf den Organismus. So hat sich überraschenderweise herausgestellt, daß dann, wenn die Windungen der Sendespule eine ovale Spirale bilden und vorteilhafterweise auf beiden Seiten einer Trägerplatte verteilt sind, sich die besten Ergebnisse erzielen lassen.

Auch muß die Sendespule in der Lage sein, die optimalen Pulsformen, -frequenzen und -leistungen störungsfrei abzustrahlen.

In allen Fällen sollte die Form der Sendespule derart sein, daß sich im bestrahlten Organismus an den Wirkungsstellen die erforderlichen Spannungen und Felder aufbauen können, ohne daß es zu gefährlichen örtlichen Feldspitzen kommt. In diesem Sinne ist die Ausgestaltung der Sendespule als sogenannter Quadrupol optimal.

Demselben Zweck dient auch eine Anpassung der Trägerplatte, die die Windungen der Sendespule trägt, an das zu behandelnde Körperteil.

Anhand der Zeichnung soll die Erfindung in Form von Ausführungsbeispielen näher erläutert werden. Es zeigen
- Fig. 1: eine Ausführung der erfindungsgemäßen Vorrichtung als fahrbares Standgerät mit einer ersten und einer zweiten Sendespule,
- Fig. 2: den Aufbau der zweiten Sendespule als Sprengbild,
- Fig. 3: einen Langsschnitt durch die erste Sendespule,
- Fig. 4: eine Draufsicht auf das Innere der ersten Sendespule,
- Fig. 5: eine Draufsicht auf eine alternative Ausführungsform der ersten Sendespule,
- Fig. 6: eine optimale Pulsform als Amplituden-Zeit-Diagramm und
- Fig. 7: die Pulse der Fig. 6 in anderem Zeitmaßstab.

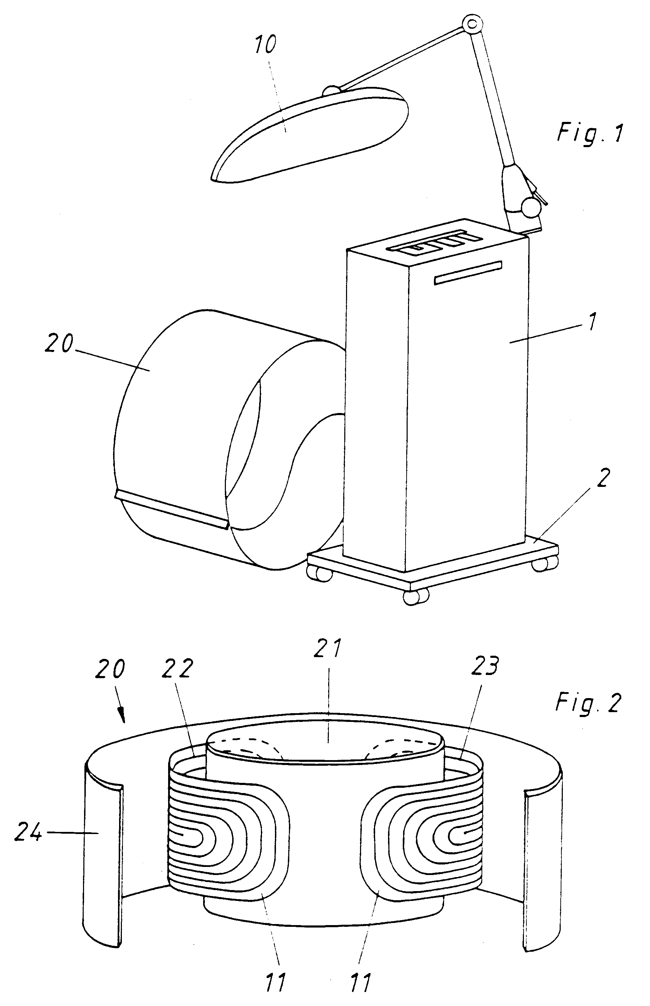

Fig. 1 zeigt ein Behandlungsgerät, wie es in der medizinischen Praxis Verwendung finden kann. Ein Grundgerät 1, welches Stromversorgung, Generator sowie Bedienteil enthält, steht auf einem Fahrwerk 2. Über einen Gelenkarm 3 ist eine erste Sendespule 10 mit dem Grundgerät 1 verbunden.

Die Sendespule 10 ist als im wesentlichen flache Platte ausgebildet. Eine zweite Sendespule 20, hier als Ring ausgebildet, ist neben dem Grundgerät 1 stehend gezeichnet.

Fig. 2 zeigt die ringförmige Sendespule 20 als Sprengbild. Zwischen einem Innenring 21 und einem Außenring 24, beide aus isolierendem, für elektromagnetische Felder durchlässigem Material bestehend, befinden sich die eigentlichen Sendewicklungen 22, 23. Jede dieser Wicklungen 22, 23 bildet eine ovale Spirale. Die Wicklungen 22, 23 können entweder auf einer oder auf beiden Seiten einer Trägerplatte 11 angebracht sein.

Zahl und Größe der Sendewicklungen 22, 23 kann in Abhängigkeit von der Größe der Sendespulen und den erforderlichen Feldstärken und Pulsleistungen variiert werden.

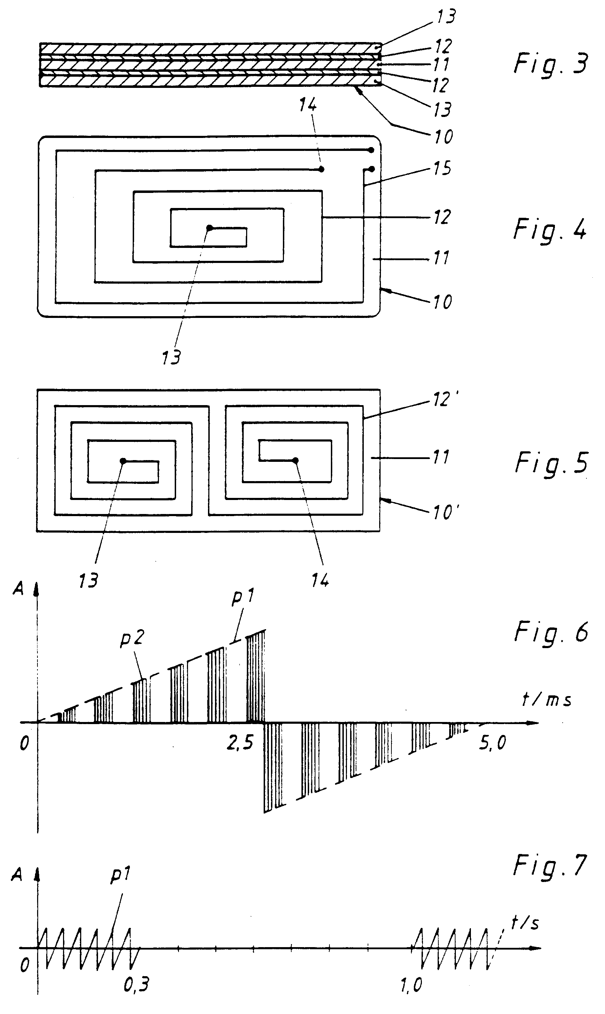

Fig. 3 zeigt einen Langsschnitt durch die flache Sendespule 10. Auch hier liegt eine Tragerplatte 11, die auf ihrer Ober- und Unterseite je eine Sendewicklung 12 tragt, zwischen einer oberen und unteren Abdeckung 13 aus isolierendem, für elektromagnetische Felder durchlässigen Material.

Fig. 4 zeigt eine Draufsicht auf eine erste Ausführungsform der Sendespule 10. Auf der Trägerplatte 11 befindet sich die eigentliche Sendewicklung 12 in Form einer ovalen Spirale, hier der Einfachheit halber mit rechteckigem Querschnitt wiedergegeben. Das innere Wicklungsende 13 ist durchkontaktiert zu einer auf der Rückseite der Tragerplatte 11 aufgebrachten weiteren Wicklung. Die Stromzuführung erfolgt am äußeren Wicklungsende 14.

Um die Sendewicklung 12 herumgelegt ist eine induktive Fühlerwicklung 15. Diese nimmt das von der Sendewicklung 12 abgestrahlte und im zu behandelnden Organismus Phasenverschoben reflektierte Feld auf und fuhrt es in das Grundgerät 1, wo die Phasenverschiebung mit einer geeigneten elektronischen Schaltung ermittelt wird. Anhand der Phasenverschiebung können die gepulsten elektromagnetischen Felder im Sinne einer optimierten biologischen Wirkung geregelt werden.

Fig. 5 zeigt eine zweite Ausführungsform der ersten Sendespule 10'. Auf der Tragerplatte 11 befinden sich zwei ovale Spiralen 12' mit gegenläufigem Wicklungssinn. Entsprechende Wicklungen befinden sich auf der Rückseite der Trägerplatte 11. Dank dieser speziellen Wicklungsform bildet sich ein sogenannter Quadropol aus, dessen Feldlinien noch besser geeignet sind, die gewünschten Effekte im Organismus zu erzielen.

Fig. 6 zeigt eine Form der Ströme, mit deren Hilfe in den Sendespulen 10, 10', 20 die gepulsten elektromagnetischen Felder erzeugt werden, die nach den bisherigen Versuchen einen optimalen Effekt im Organismus erzeugen. Die Amplitude A des Grundpulses p1 stellt ein symmetrisches Dreieck dar, dessen Polarität sich nach der halben Pulszeit umkehrt. Die Amplitude steigt langsam an und fällt ebenso langsam ab. Eingelagert in den Grundpuls p1, dessen Grundfrequenz f1 im vorliegenden Beispiel 200 Hz beträgt, sind Impulszüge p2 einer erhöhten Frequenz f2 von ca. 10 kHz. Die Grundfrequenz f1 ist auf die mechanische Resonanz der Blut- und Lymphgefäße abgestimmt. Die erhöhte Frequenz f2 ist auf die kapazitive Überleitung durch die Gefäßwände und Membranen hindurch abgestimmt. Von wesentlicher Bedeutung ist, daß die Pulsamplitude so stark gewählt wird, daß im behandelnden Organismus die nötigen Spannungen und Feldstarken induziert werden.

Fig. 7 zeigt die Grundpulse p1 in einem Diagramm mit geänderter Zeitachse. Die Grundpulse p1, denen wie in der Fig. 6 dargestellt die 10 kHz-Pulse p2 überlagert sind, bleiben für 0,3 sec. eingeschaltet. Anschließend werden sie für eine Pause von 0,7 sec. unterbrochen, um dann für weitere 0,3 sec. eingeschaltet zu werden usw. Die dargestellten Ein-Aus-Zeiten von 0,3 sec. : 0,7 sec. haben sich bei den Versuchen als guter Mittelwert herausgestellt. Die Feinabstimmung kann in Abhängigkeit von der Wirkung auf den behandelnden Organismus variiert werden. Hierzu dient entweder die induktive Fühlerwicklung 15 (Fig. 4) oder ein anderer geeigneter Aufnehmer, beispielsweise ein Blutdruckmeßgerät, ein Thermograph oder ein Pulsmeßgerät.

Wie die Versuche gezeigt haben, sind Form und Konstruktion der Sendespulen von besonderer Bedeutung für die optimale Wirkung des erfindungsgemäßen Gerätes. Obwohl die Grundfrequenz mit 50 bis 500 Hz, vorzugsweise 200 Hz, sehr niedrig ist, treten durch das Ein- und Ausschalten der 10 kHz-Pulse p2 und den Polaritätswechsel hochfrequente Oberwellen auf. Bei einer nicht-optimierten Spulenform werden die Oberwellen nicht ausreichend übertragen, d. h. die Impulsform wird verändert und die Wirkung verschlechtert sich. Auch müssen durch die Formgebung der Sendespulen etwaige örtliche Feldlinienkonzentrationen verhindert werden, die zu einer Schädigung des Organismus Anlaß sein könnten.

Wie die Versuche gezeigt haben, lassen sich grundsätzlich alle biologischen Organismen behandeln. Organismen mit ausgebildetem Blut- oder Lymphkreislauf, d. h. Menschen und Säugetiere, sind dabei bevorzugt. Mit Hilfe des erfindungsgemäßen Gerätes können beispielsweise bei Reit- und Springpferden die Muskelbildung und die Gelenkregeneration, bei Kühen die Milcherzeugung und bei Schweinen die Fleischbildung angeregt werden. Bei der Behandlung von Menschen ist die Verwendung des Gerätes in der Medizin und im Sport angezeigt.

## Patentansprüche

1. Vorrichtung zum Bewirken des Transports von Protonen aus intrakorporalen Elektrolytflüssigkeiten in die umgebenden Gefäßwände und Membranen, bestehend aus einem Grundgerät (1) mit einem Generator zur Erzeugung elektromagnetischer Pulse (p1, p2) und einer Sendespule (10, 10', 20), wobei die Grundfrequenz (f1) der Grundpulse (p1) zwischen 50 und 500 Hz, vorzugsweise bei 200 Hz liegt, die Amplitude (A) jedes Grundpulses (p1) langsam ansteigt und abfällt, die Polarität der Amplitude (A) der Grundpulse (p1) wenigstens einmal wechselt, die Sendespule (10, 10', 20) als Flachspule ausgebildet ist, deren Windungen (12, 12', 22, 23) auf der Oberfläche einer Trägerplatte (11) angebracht sind, die Sendeenergie so hoch ist, daß in der Elektrolyt-Flüssigkeit eine Spannung induziert wird, die höher ist als die thermische Spannung, wobei die Zetapotential genannte Potentialschwelle, die durch die stark differierenden relativen Dielektrizitätskonstanten der Gefäßwände einerseits und der Elektrolytflüssigkeiten andererseits entsteht, neutralisiert werden kann.

2. Vorrichtung nach Anspruch 1, dadurch gekennzeichnet, daß die Grundpulse (p1) nach jeder halben Pulszeit die Polarität wechseln.

3. Vorrichtung nach Anspruch 1 oder 2, dadurch gekennzeichnet, daß die Amplitude (A) der Grundpulse (p1) ein symmetrisches Dreieck bildet.

4. Vorrichtung nach Anspruch 1, 2 oder 3, dadurch gekennzeichnet, daß jedem Grundpuls (p1) Pulspakete (p2) einer erhöhten Frequenz von ca. 10 KHz überlagert sind.

5. Vorrichtung nach einem der Ansprüche 1 bis 4, dadurch gekennzeichnet, daß die Grundpulse (p1) in regelmäßigen Folgen ein- und ausgeschaltet sind, wobei die Ein- und Ausschaltzeiten bei ca. 0,3 sec. : 0,7 sec. liegen.

6. Vorrichtung nach Anspruch 5, dadurch gekennzeichnet, daß die Ein/Aus-Zeiten mittels Biofeedback variierbar sind.

7. Vorrichtung nach einem der Ansprüche 1 bis 6, dadurch gekennzeichnet, daß ein Blutdruckmeßgerät und/oder ein Pulsmeßgerät anschließbar ist.

8. Vorrichtung nach einem der Ansprüche 1 bis 7, dadurch gekennzeichnet, daß ein Thermograph anschließbar ist.

9. Vorrichtung nach einem der Ansprüche 1 bis 8, dadurch gekennzeichnet, daß eine induktive Fühlerwicklung (15) anschließbar ist.

10. Vorrichtung nach Anspruch 9, dadurch gekennzeichnet, daß die Fühlerwicklung (15) mit der Sendespule (12, 12', 22, 23) auf derselben Trägerplatte (11) angebracht ist.

11. Vorrichtung nach einem der Ansprüche 1 bis 10, dadurch gekennzeichnet, daß die Windungen (12, 12', 22, 23) der Sendespule (10, 10', 20) eine ovale Spirale bilden.

12. Vorrichtung nach einem der Ansprüche 1 bis 11, dadurch gekennzeichnet, daß die Windungen (12, 12', 22, 23) der Sendespule auf beiden Seiten der Trägerplatte (11) verteilt sind.

13. Vorrichtung nach einem der Ansprüche 1 bis 12, dadurch gekennzeichnet, daß die Windungen (12') der Sendespule (10') einen Quadrupol bilden.

14. Vorrichtung nach einem der Ansprüche 1 bis 13, dadurch gekennzeichnet, daß der Generator bezüglich Frequenz (f1, f2), Amplitude (A), Kurvenform (p1, p2) bzw. Ein/Aus-Zeiten abstimmbar ist.

## Claims

1. A device for causing protons to migrate out of electrolytic internal body fluids into the surrounding vessel walls and membranes, comprising a basic apparatus (1) with a generator for generating electromagnetic pulses (p1,p2) and an emitter coil (10,10',20), wherein the basic frequency (f1) of the basic pulses (p1) lies between 50 and 500 Hz, preferably at 200 Hz, the amplitude (A) of each basic pulse (p1) increases and decreases slowly, the polarity of the amplitude (A) of each basic pulse (p1) changes at least once, the emitter coil (10,10',20) is in the form of a flat coil whose windings (12,12',22,23) are mounted on the surface of a supporting plate (11), the emitter energy being so high that in the electrolytic fluid a voltage is induced which is higher than the thermionic potential, wherein said potential threshold, known as the zeta potential, which occurs as a result of the greatly differing relative dielectric constants of the vessel walls on the one hand and the electrolytic fluid on the other hand, can be neutralised.

2. A device according to claim 1, characterised in that the polarity of the basic pulses (p1) changes after every half pulse period.

3. A device according to Claim 1 or 2, characterised in that the amplitude (A) of the basic pulses (p1) forms a symmetrical triangle.

4. A device according to Claim 1, 2 or 3, characterised in that pulse packets (p2) of a high frequency of approximately 10 KHz are superimposed on each basic pulse (p1).

5. A device according to any one of Claims 1 to 4, characterised in that the basic pulses (p1) are switch on and off in regular succession, wherein the switching-on and switching-off times are approximately 0.3 sec. : 0.7 sec.

6. A device according to Claim 5, characterised in that the on/off times can be varied by means of biofeedback.

7. A device according to any one of Claims 1 to 6, characterised in that a blood-pressure measurement apparatus and/or a pulse measurement apparatus can be connected.

8. A device according to any one of Claims 1 to 7, characterised in that a thermograph can be connected.

9. A device according to any one of Claims 1 to 8, characterised in that an inductive sensor winding (15) is connected.

10. A device according to Claim 9, characterised in that the sensor winding (15) is mounted with the emitter coil (12,12',22,23) on the same supporting plate (11).

11. A device according to any one of Claims 1 to 10, characterised in that the windings (12,12',22,23) of the emitter coil (10,10',20) form an oval spiral.

12. A device according to any one of Claims 1 to 11, characterised in that the windings (12,12',22,23) of the emitter coil are spaced apart on either side of the supporting plate (11).

13. A device according to any one of Claims 1 to 12, characterised in that the windings (12,12',22,23) of the emitter coil (10') form a quadrupole.

14. A device according to any one of Claims 1 to 13, characterised in that the generator can be tuned with respect to frequency (f1,f2), amplitude (A), curve shape (p1,p2) and/or on/off times.

## Revendications

1. Dispositif pour opérer le transport de protons issus de liquides électrolytiques intracorporels dans les parois vasculaires et membranes environnantes, comportant un appareil de base (1) avec un générateur permettant de générer des impulsions électromagnétiques (p1, p2) et une bobine émettrice (10, 10', 20), la fréquence de base (f1) des impulsions de base (p1) se situant entre 50 et 500 Hz et étant de préférence de 200 Hz, l'amplitude (A) de chaque impulsion de base (p1) croissant et décroissant lentement, la polarité de l'amplitude (A) de l'impulsion de base alternant au moins une fois, la bobine émettrice (10, 10', 20) étant conçue en tant que bobine plate dont les spires (12, 12', 22, 23) sont placées sur la surface d'une plaque support (11), l'énergie d'émission étant suffisamment élevée pour induire dans le liquide électrolytique une tension qui est plus élevée que la tension thermique, le seuil de potentiel appelé potentiel zêta qui se forme par les constantes diélectriques relatives fortement différentes d'une part, des parois vasculaires et d'autre part, des liquides électrolytiques d'autre part, pouvant être neutralisé.

2. Dispositif selon la revendication 1, caractérisé en ce que les impulsions de base (p1) changent de polarité à la moitié de la durée d'impulsion.

3. Dispositif selon la revendication 1 ou 2, caractérisé en ce que l'amplitude (A) des impulsions de base (p1) forme un triangle symétrique.

4. Dispositif selon la revendication 1, 2 ou 3, caractérisé en ce que des trains d'impulsions (p2) d'une fréquence élevée de 10 KHz environ se superposent à chaque impulsion de base (p1).

5. Dispositif selon l'une quelconque des revendications 1 à 4, caractérisé en ce que les impulsions de base (p1) sont déclenchées et arrêtées en séquences régulières, les temps de déclenchement et d'arrêt étant de 0,3 s. à 0,7 s. environ.

6. Dispositif selon la revendication 5, caractérisé en ce que les temps de déclenchement/ d'arrêt peuvent varier à l'aide d'un bio-feedback.

7. Dispositif selon l'une quelconque des revendications 1 à 6, caractérisé en ce qu'il est possible de brancher un appareil de mesure de la pression sanguine et/ou un appareil de mesure des pulsations.

8. Dispositif selon l'une quelconque des revendications 1 à 7, caractérisé en ce qu'il est possible de brancher un thermographe.

9. Dispositif selon l'une quelconque des revendications 1 à 8, caractérisé en ce qu'il est possible de raccorder un enroulement inductif à capteur (15).

10. Dispositif selon la revendication 9, caractérisé en ce que l'enroulement à capteur (15) est fixé avec la bobine d'émission (12, 12', 22, 23) sur la même plaque support (11).

11. Dispositif selon l'une des revendications 1 à 10, caractérisé en ce que les spires (12, 12', 22, 23) de la bobine d'émission (10, 10', 20) forment une spirale ovale.

12. Dispositif selon l'une des revendications 1 à 11, caractérisé en ce que les spires (12, 12', 22, 23) de la bobine d'émission sont réparties des deux côtés de la plaque support (11).

13. Dispositif selon l'une des revendications 1 à 12, caractérisé en ce que les spires (12') de la bobine d'émission (10') forment un quadripôle.

14. Dispositif selon l'une des revendications 1 à 13, caractérisé en ce que le générateur peut être réglé, en ce qui concerne la fréquence (f1, f2), l'amplitude (A), l'allure de la courbe (p1, p2) et les temps de déclenchement/ d'arrêt.
